# EUROPEAN PATENT SPECIFICATION

(11) **EP 0 912 150 B1**
(45) Date of publication and mention of the grant of the patent: **02.10.2002**
(21) Application number: 97928944.4
(22) Date of filing: 10.06.1997
(51) Int. Cl.: A61F 13/15

(54) **ABSORBENT ARTICLES HAVING PLEATED UNDERGARMENT COVERING COMPONENTS**
ABSORBIERENDE ARTIKEL MIT GEFALTETEN WÄSCHESCHUTZELEMENTEN
ARTICLES ABSORBANTS COMPORTANT DES ELEMENTS PLISSES RECOUVRANT LES SOUS-VETEMENTS

(30) Priority: 11.06.1996 US 661683
(43) Date of publication of application: 06.05.1999
(73) Proprietor: THE PROCTER & GAMBLE COMPANY, Cincinnati, Ohio 45202 (US)
(72) Inventor: WEINBERGER, Eric, Patton, Fairfield, OH 45014 (US)
(74) Representative: Kremer, Véronique
(86) International application number: US9710089
(87) International publication number: WO97047266

(56) References cited:
- EP-A- 0 426 235
- EP-A- 0 695 542
- WO-A-93/06805
- WO-A-94/12135
- WO-A-96/10977

## Description

The present invention relates to absorbent articles such as sanitary napkins, panty liners, and incontinence pads. More particularly, the present invention relates to sanitary napkins that have either side flaps, or undergarment covering components (or "side wrapping elements"), the latter preferably automatically wrap the sides of a wearer's undergarments when the undergarments are pulled up, and serve as an alternative to conventional side flaps.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins both with and without side flaps (or wings) are disclosed in the literature and are available in the marketplace.

Generally when sanitary napkins are provided with flaps, the flaps extend laterally from a central absorbent means and are intended to be folded around the edges of the wearer's panties in the crotch region. Commonly, the flaps are provided with an attachment means for either affixing the flaps to the underside of the wearer's panties or to the opposing flap. The flaps are particularly effective for preventing exudates from soiling the edges of the wearer's panties.

Sanitary napkins having flaps of various types are disclosed in U.S. Patent 4,687,478, entitled "Shaped Sanitary Napkin With Flaps", which issued to Van Tilburg on August 18, 1987; U.S. Patent 4,608,047, entitled "Sanitary Napkin Attachment Means", which issued to Mattingly on August 26, 1986; U.S. Patent 4,589,876, entitled "Sanitary Napkin", which issued to Van Tilburg on May 20, 1986 and its Reexamination Patent No. B1 4,589,876, Certificate of Reexamination issued April 27, 1993; U.S. Patent 4,285,343, entitled "Sanitary Napkin", which issued to McNair on August 25, 1981; U.S. Patent 3,397,697, entitled "Disposable Sanitary Shield For Undergarments", which issued to Rickard on August 20, 1968; and, U.S. Patent 2,787,271, entitled "Sanitary Napkin", which issued to Clark on April 2, 1957.

Several variations of sanitary napkins having conventional flaps are also disclosed in the patent literature. For example, U.S. Patent 4,911,701 issued to Mavinkurve discloses a sanitary napkin having elastic strands for providing a greater convex shape to the body-facing portion of the central absorbent and for enabling adhesive-free placement of the flaps of a winged napkin embodiment into a pair of panties. U.S. Patent 4,940,462 issued to Salerno discloses a sanitary napkin with longitudinally expandable flaps. The flaps are designed to fold over the exterior of the wearer's panty and then to expand to conform with the contour of the panties. The Salerno patent, however, appears to require conventional adhesive fasteners to retain the flaps in place on the underside of the wearer's panties.

Another drawback to the flap construction shown in both the Mavinkurve and Salerno references is that the attachment of the expandable flaps directly to the longitudinal sides of the absorbent element leads to the problem that any compression of the absorbent element will cause the flaps to retract transversely inward. Any bunching of the absorbent element in the sanitary napkins shown in these references, such as that caused by compression by the wearer's legs, causes the flaps to lose their ability to cover a given area of the wearer's panties.

Several sanitary napkins are disclosed in the literature that are provided with an alternative to conventional flaps that automatically wrap around the sides of the wearer's panties by the simple action of the wearer pulling up her panties. These latter types of sanitary napkins are described in PCT Publication No. WO 94/02096, entitled "Absorbent Articles Having Panty Covering Components That Naturally Wrap The Sides of Panties", published in the name of Lavash, et al., on February 3, 1994, and in PCT Publication No. WO 94/03025, entitled "Absorbent Articles Having Undergarment Covering Components With Zones of Extensibility", published in the name of Weinberger, et al., on February 2, 1995. Sanitary napkins having improved structures for side flaps and side wrapping elements are also described in WO 94/12135, EP 695542, and in WO 96/10977.

While the sanitary napkins described in the Lavash patent, the Van Tilburg patents, and the PCT and EP publications mentioned above work well, the search for improvements to the structures described therein has continued. For example, it has been found that there is occasionally a tendency for portions, particularly at the ends of the side wrapping elements on these sanitary napkins to flip upward and fold over the topsheet of the sanitary napkin in response to forces exerted thereon by the wearer's panty elastics. This can result in discomfort to the wearer, and may cause the side wrapping elements to become displaced from their intended wrapped position. It has also been found that if the flaps or side wrapping elements are provided with zones of extensibility, it is desirable for the extensibility to begin underneath the main body portion of the absorbent article so that the flaps or side wrapping elements will properly adjust to fit a variety of different size and style panties. This latter task has been found to be a particularly difficult one when the flaps or side wrapping elements are integral extensions of components of the main body portion of the absorbent article, such as the topsheet of the absorbent article, the backsheet, or both.

Thus, a need exists for an absorbent article, such as a sanitary napkin, that is provided with side flaps or an alternative to conventional flaps that reduces the tendency for the ends of the flaps or side wrapping elements to flip upward and fold over the topsheet of the sanitary napkin.

It is, therefore, an object of the present invention to provide an absorbent article, such as a sanitary napkin, that is provided with an improved stzucture that reduces the tendency for the ends of such side wrapping elements to flip upward and fold over the topsheet of the sanitary napkin.

It is another object of the present invention to provide an absorbent article, such as a sanitary napkin, that has flaps with zones of differential extensibility therein which is located beneath at least a portion of the absorbent core of the absorbent article when the flaps are integral extensions of components of the main body portion of the absorbent article.

It is still another object of the present invention to provide an absorbent article, such as a sanitary napkin, that has an alternative means for reducing the undesirable effects on the flaps or side wrapping elements when the absorbent core of the absorbent article is compressed by the wearer's legs.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin. The sanitary napkin of the present invention has either a pair of side flaps, or undergarment covering components (or "side wrapping elements") that provide coverage to the wearer's panties to reduce side soiling (i.e., staining of the edges of the panty crotch).

The sanitary napkin comprises a main body portion comprising a liquid pervious topsheet, a liquid impervious backsheet joined to the topsheet, and an absorbent core positioned between the topsheet and the backsheet. The flaps or side wrapping elements preferably comprise integral extensions of at least one of the topsheet and the backsheet. The flaps or side wrapping elements comprise a pair of flexible elements that extend beyond the crotch edge portions of the wearer's undergarments. The flaps or side wrapping elements are preferably each provided with at least one zone of extensibility, preferably two spaced apart zones of extensibility. The zones of extensibility are regions of the side wrapping elements that have a greater range of extension than the adjacent regions of the side wrapping elements. The flaps or side wrapping elements preferably comprise at least one zone of extensibility on each side of the transverse centerline of the flaps or side wrapping elements and a stiffer, less extensible intermediate region along the transverse centerline of the flaps or side wrapping elements. The extension of the topsheet, the backsheet, or both that forms the flaps or side wrapping elements is pleated and attached to the garment-facing side of the main body portion.

More specifically, the flaps or side wrapping elements are folded under the garment-facing side of the main body portion of the sanitary napkin toward the longitudinal centerline of the sanitary napkin. The folded portion is secured to the garment-facing side of the main body portion at a point laterally inward from the longitudinal side edges of the main body portion. The flaps or side wrapping elements are folded laterally back outward away from the longitudinal centerline, and freely extend outwardly therefrom, except in at least one region, and preferably two regions, on each flap or side wrapping element. In at least one region, portions at the ends of the flaps or side wrapping elements are secured to the garment-facing side of the main body portion. The securement of portions of the ends of the flaps or side wrapping elements in this manner, and the folding and attachment of the same under the absorbent core, beneath the plane of the body-facing side of the main body portion, helps prevent the ends of the flaps or side wrapping elements from flipping upward over the topsheet and causing discomfort for the wearer and/or loss of coverage of the wearer's undergarment.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:

FIG. 1 is a top plan view of the sanitary napkin of the present invention having side flaps.

FIG. 2 is a cross-sectional view of the sanitary napkin shown in FIG. 1 taken along line 2-2 of FIG. 1.

FIG. 3 is a modified bottom plan view of the sanitary napkin shown in FIG. 1 with a portion of the release paper therein peeled back to show the underlying structure.

FIG. 4 is a top plan view of a sanitary napkin of the present invention which has side wrapping elements that automatically fold around the sides of a wearer's undergarments instead of side flaps.

FIG. 5 is a top plan view of a sanitary napkin according to the present invention which has side wrapping elements of the sanitary napkin in an alternative configuration.

### DETAILED DESCRIPTION OF THE INVENTION

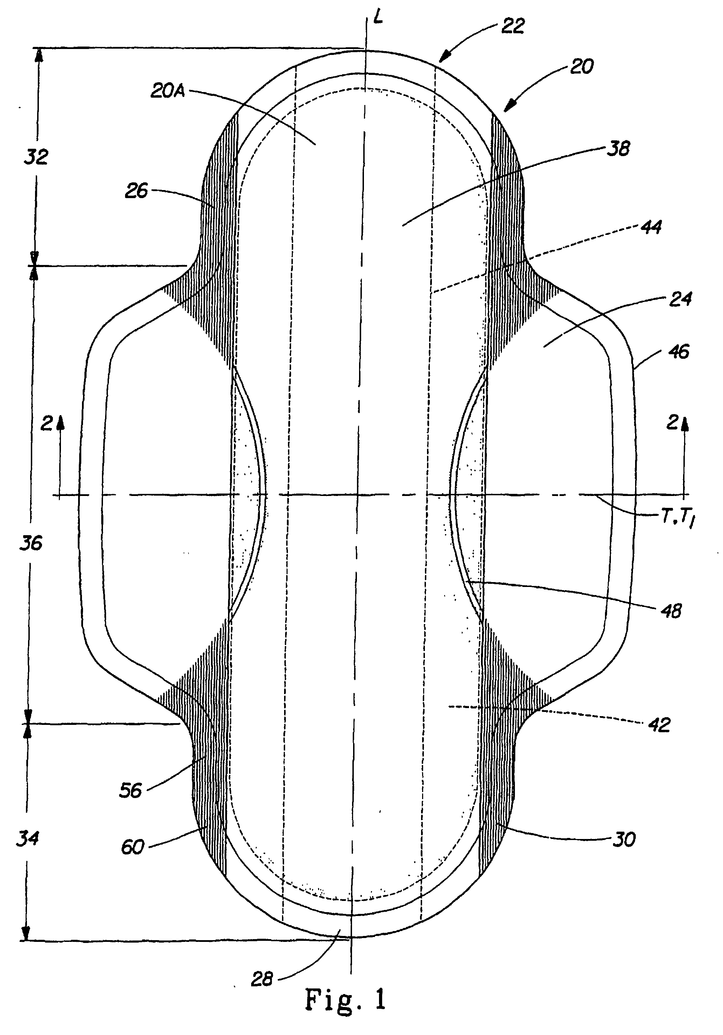

FIGS. 1-3 show one preferred embodiment of a disposable absorbent article of the present invention 20. The present invention relates to absorbent articles, such as sanitary napkins, pantiliners, and incontinence pads. The sanitary napkin 20 shown in FIGS. 1-3 has a main body portion 22 and a pair of side flaps 24 for folding around the sides of a wearer's panties.

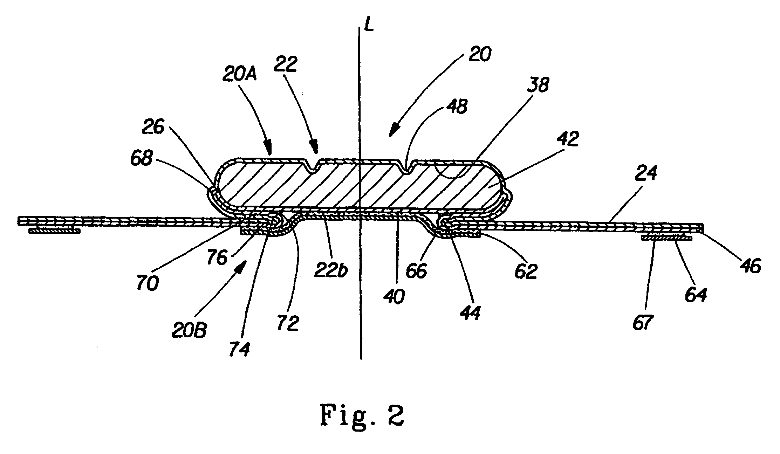

The sanitary napkin 20 has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 (shown in FIG. 2) is on the opposite side and is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows that the main body portion 22 of the sanitary napkin 20 comprises the portion of the sanitary napkin without the flaps 24. The main body portion 22 has two spaced apart longitudinal edges 26, two spaced apart transverse or end edges (or "ends") 28, which together form the periphery 30 of the main body portion. The main body portion also has two end regions, which are designated first end region 32 and second end region 34. A central region 36 is disposed between the end regions 32 and 34. The end regions 32 and 34 extend outwardly from the edges of the central region 36 about 1/8 to about 1/3 of the length of the main body portion. A detailed description of a sanitary napkin having a central region 36 and the two end regions 32 and 34 is contained in U.S. Patent 4,690,680 issued to Higgins on September 1, 1987.

The main body portion 22 of the sanitary napkin 20 can be of any thickness, including relatively thick, intermediate thickness, relatively thin, or even very thin or "ultra thin". An "ultra-thin" sanitary napkin 20 as described in U.S. Patents 4,950,264 and 5,009,653 issued to Osborn preferably has a caliper of less than about 3 millimeters. The embodiment of the sanitary napkin 20 shown in Figures 1-3 of the drawings is intended to be an example of a sanitary napkin having a main body portion 22 of intermediate thickness. It should be understood that the sanitary napkin shown is merely one preferred embodiment, and that is not limited to absorbent articles of the type or having the specific configurations shown in the drawings.

FIG. 2 shows the individual components of the main body portion 22 of the sanitary napkin 20. The main body portion 22 of the sanitary napkin generally comprises at least three primary components. These include a liquid pervious topsheet 38, a liquid impervious backsheet 40, and an absorbent core 42 positioned between the topsheet 38 and the backsheet 40. The main body portion 22 may also include any other optional components known in the art. For example, the sanitary napkin 20 can include a wipe acquisition sheet such as that described in the aforementioned Osborn patents positioned between the topsheet 38 and the absorbent core 42. Alternatively, or additionally, the sanitary napkin 20 could be provided with an optional tissue layer between the absorbent core 42 and the backsheet 40. Numerous other embodiments are possible. The topsheet, the backsheet, and the absorbent core may be assembled in a variety of configurations known in the art (including so called "sandwich" products and "tube" products).

Several preferred sanitary napkin configurations are described generally in U.S. Patent 4,321,924, "Bordered Disposable Absorbent Article" issued to Ahr on March 30, 1982; U.S. Patent 4,425,130, "Compound Sanitary Napkin" issued to DesMarais on January 10, 1984; U.S. Patent 4,950,264, "Thin, Flexible Sanitary Napkin" issued to Osborn on August 21, 1990; U.S. Patent 5,308,346, "Elasticized Sanitary Napkin" issued to Sneller, et al. on May 3, 1994; allowed U.S. Patent Application Serial No. 08/096,121 entitled "Absorbent Articles Having Panty Covering Components That Naturally Wrap the Sides of Panties" filed July 22, 1993, in the name of Lavash, et al.; and U.S. Patent Application Serial No. 08/124,180 entitled "Absorbent Articles Having Panty Covering Components Comprising Extensible Web Materials Which Exhibit Elastic-Like Behavior" filed September 17, 1993, in the name of Mansfield, et al. The main body portion 22 of the sanitary napkin may also be comprised of one or more extensible components such as those sanitary napkins, and the like described in U.S. Patent Application Serial Nos. 07/915,133 and 07/915,284 both filed July 23, 1992, in the name of Osborn, et al., a continuation of the latter application is currently pending as allowed U.S. Patent Application Serial No. 08/503,895, filed July 18, 1995 (the original applications corresponding to PCT Publication Nos. WO 93/01785 and 93/01786, both published February 4, 1993).

Figures 1-3 show a preferred embodiment of the sanitary napkin 20 assembled in a sandwich construction in which the topsheet 38 and the backsheet 40 have length and width dimensions generally larger than those of the absorbent core 42. The main body portion 22 of the sanitary napkin shown in FIGS. 1-3 is preferably provided with a pair of embossed channels 48 similar to those described in U.S. Patent 5,308,346 issued to Sneller, et al. The topsheet 38 and the backsheet 40 extend beyond the edges of the absorbent core 42 to thereby form portions of the periphery 30. The sanitary napkin 20 of the present invention comprises a pair of side flaps 24 that extend laterally outward beyond the longitudinal side edges 26 of the main body portion 22 from their proximal edges 44 to their distal edges 46.

The side flaps 24 are preferably integral extensions of the topsheet 38 and backsheet 40 of the main body portion 22. The side flaps 24 can, however, be joined to the main body portion 22 in any suitable manner. The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. Thus, in other embodiments, the side flaps 24 can, comprise two separate components that are joined to the garment-facing side of the main body portion 22. In still other embodiments, instead of comprising two separate components, the side flaps 24 can comprise a single component, or each side flap 24 can comprise more than one component.

Since the side flaps 24 are preferably integral extensions of portions of the main body portion 22 of the sanitary napkin 20, the side flaps 24, can be made from any of the materials used in the construction of the main body portion 22 of the sanitary napkin. The side flaps 24 in the embodiment shown in FIGS. 1-3 preferably comprise a laminate of two materials, three dimensional formed film, and a liquid impervious backing such as a polyethylene film backsheet material. The three dimensional formed film is preferably the apertured film known as DRI-WEAVE which is used as a topsheet on sanitary napkins manufactured by the Procter & Gamble Company, Cincinnati, Ohio under U.S. Patents 4,342,314 issued to Radel, et al. and 4,463,045 issued to Ahr, et al. The polyethylene backsheet material is a film comprised of LLDPE that preferably has a caliper of between about 0.4-1.5 mil, more preferably about 1 mil. A preferred polyethylene film is known as #P18-1401 and is obtained from Ciopay Corporation of Cincinnati, Ohio. The polyethylene film can be joined to the apertured formed film in any suitable manner. Preferably, the polyethylene film is glued to the apertured formed film such as by a spiral application of adhesives.

The side flaps 24 have a flap transverse centerline T₁. The side flaps 24 can be in any suitable configuration. Several preferred configurations for the side flaps 24 are described in U.S. Patent 4,589,876, entitled "Sanitary Napkin", which issued to Van Tilburg on May 20, 1986 and its Reexamination Patent No. B1 4,589,876, Certificate of Reexamination issued April 27, 1993; U.S. Patent 4,687,478, entitled "Shaped Sanitary Napkin With Flaps", which issued to Van Tilburg on August 18, 1987; U.S. Patent 5,281,209, entitled "Absorbent Article Having Tucked Flaps", which issued to Osborn, et al. on January 25, 1994; U.S. Patent 4,344,416, entitled "Absorbent Article Having Inwardly-Folded Pleated Flaps", which issued to Niihara on September 6, 1994; and U.S. Patent 5,389,494, entitled "Absorbent Article Having Flaps and Zones of Differential Extensibility", which issued to Lavash, et al. on February 14, 1995. In a particularly preferred embodiment, the side flaps 24 are provided with zones of differential extensibility as described in the Lavash, et al. patent.

FIGS. 1-3 show that the flaps 24 are folded along a first fold (or first fold line) 68 under the garment-facing side 22B of the main body portion 22 of the sanitary napkin toward the longitudinal centerline of the sanitary napkin. FIG. 2 shows that the folded portion 70 is joined to the garment-facing side 22B of the main body portion 22 at points in a region which will be referred to as the first joined area (preferably along a line) located laterally inward from the longitudinal side edges 26 of the main body portion 22. The side flaps 24 preferably are folded so that they have a gap between their proximal edges 44 of between about 30 - 60 mm, and more preferably, between about 40-50 mm. The first folded portion 70 can be joined to the garment-facing side 22B of the main body portion 22 in any suitable manner, such as by an adhesive 72. The flaps 24 are folded laterally back outward away from the longitudinal centerline, L, along a second fold (or second fold line) 74 and the second folded portions 76 freely extend outwardly therefrom, except in at least one region, and preferably two regions, on each side flap 24.

In at least one region, the ends 80 of the flaps 24 are joined to the garment-facing side 20B of the main body portion 22. The longitudinal end regions (or "ends") of the flaps 24 are the regions of the flaps 24 that are spaced longitudinally away from the flap transverse centerline, and are positioned directly below the longitudinal side edges 26 of the main body portion 22 when the flaps 24 are extended. The ends 80 of the side flaps 24 can be joined to the garment-facing side of the main body portion 22 by any suitable attachment mechanism. Suitable attachment mechanisms include, but are not limited to adhesives, and the like. In the embodiment shown, the ends 80 of the flaps 24 are joined to the main body portion 22 by the heat and/or pressure process used to form the perimeter seal (or crimp seal) around the periphery of the sanitary napkin, or around a portion thereof, such as at the ends of the main body portion 22. The flaps 24 extend from underneath the absorbent core 42 (below the plane of the topsheet). The side flaps 24 are otherwise unattached to the garment-facing side 20B of the main body portion 22 of the sanitary napkin 20 between the points of attachment and the longitudinal side edges 26 of the main body portion.

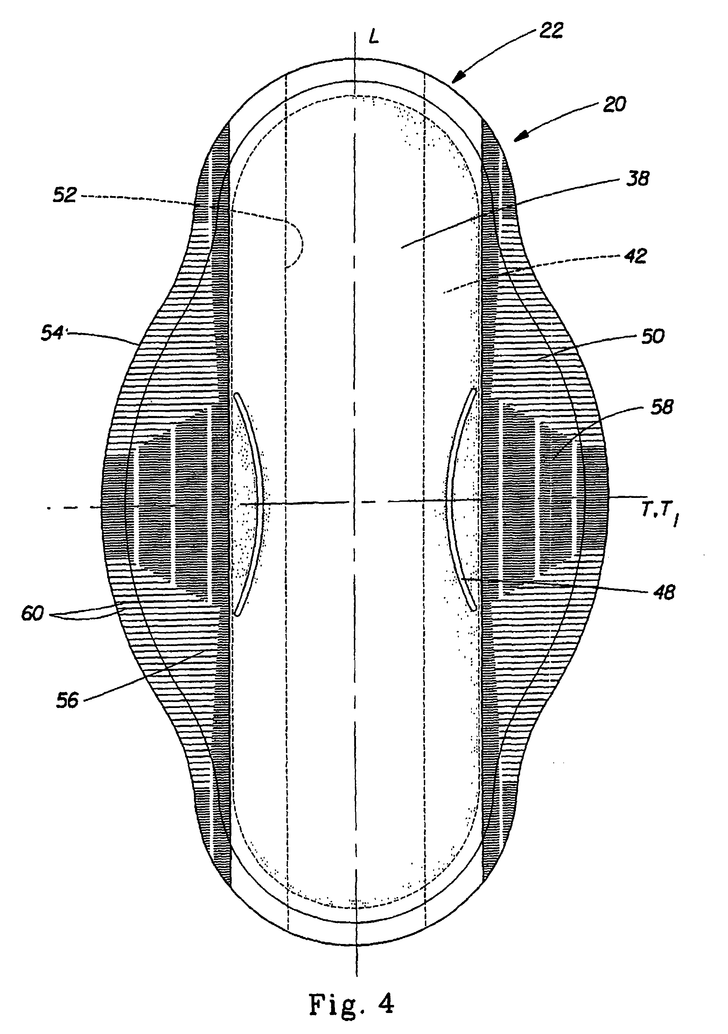

In the sanitary napkin 20 shown in FIGS. 1-3, the side flaps 24 each have at least one, and preferably two zones of extensibility 56 therein. The zones of extensibility 56, as shown in FIGS. 1-3, can be primarily extensible in the transverse direction (that is, they are extensible more in the transverse direction than in the longitudinal direction). In other embodiments, as shown in FIG. 4, the zones of extensibility 56 can be primarily extensible in the longitudinal direction. In other embodiments, the zones of extensibility 56 can be extensible in any direction between the longitudinal direction and the transverse direction, or in more than one direction. The extensibility of all the zones of extensibility 56 on the side flaps 24 can be in the same direction. Alternatively, one or more of the zones of extensibility 56 may be extensible in a different direction.

The zones of extensibility 56 in embodiments having side flaps are preferably capable of extending between about 40% and about 150%, more preferably between about 70% and about 120%, and most preferably between about 90% and about 100% for embodiments that are primarily extensible in the transverse direction, and about 50% for embodiments that are extensible in a direction between the longitudinal and transverse directions under the forces associated with wearing the sanitary napkin in a pair of panties. Preferably, the zones of extensibility 56 are capable of such extension under forces of less than about 100 - 200 grams_{f} per inch (about 40-80 gf/cm), more preferably under forces of less than about 50 grams_{f} per inch (about 20 gf/cm). The zones of extensibility 56 are also preferably extensible without being elasticized or elasticated (where separate elastic bands are stretched and attached to the side wrapping elements 50 in an extensible condition).

The side flaps 24 can be provided with zones of extensibility 56 in a nonlimiting number of different manners. The side flaps 24 may, for example, comprise a substantially inextensible material that is provided with extensible regions for the zones of extensibility. The extensible regions can be created in any suitable manner, including but not limited to mechanically straining, corrugating, "ring rolling", heating and deforming, subjecting portions of the side flaps 24 to compression between mating plates, and the like.

The embodiments shown in FIGS. 1-3 have zones of extensibility 56 formed by ring rolling (or pre-corrugating) two regions of the side flaps 24. The term "ring rolling" refers to a straining/activation achieved by feeding the material comprising the side flaps 24 between a pair of corrugated rolls having intermeshing teeth. Suitable methods for ring rolling are described in U.S. Patent 4,107,364 issued to Sisson on August 15, 1978, U.S. Patent 4,834,741 issued to Sabee on May 30, 1989, U.S. Patent 5,143,679 issued to Gerald M. Weber, et al. on September 1, 1992, U.S. Patent 5,156,793 issued to Kenneth B. Buell, et al. on October 20, 1992, and U.S. Patent 5,167,897 issued to Gerald M. Weber, et al. on December 1, 1992.

The side flaps 24 in the embodiment shown in FIGS. 1-3 are provided with ring rolled corrugations having fold lines 60 that are generally parallel to the longitudinal centerline L. The fold lines 60 can form any angle with the longitudinal centerline, between greater than or equal to 0° and less than or equal to 180°. For example, in alternative embodiments, the ring rolling can be applied so that the fold lines 60 form an angle of between about 40° - 45° with the longitudinal centerline L. In other alternative embodiments, the ring rolling can be applied so that the fold lines 60 in the corrugations are oriented generally in the transverse direction to provide zones of extensibility 56 that are primarily extensible in the longitudinal direction.

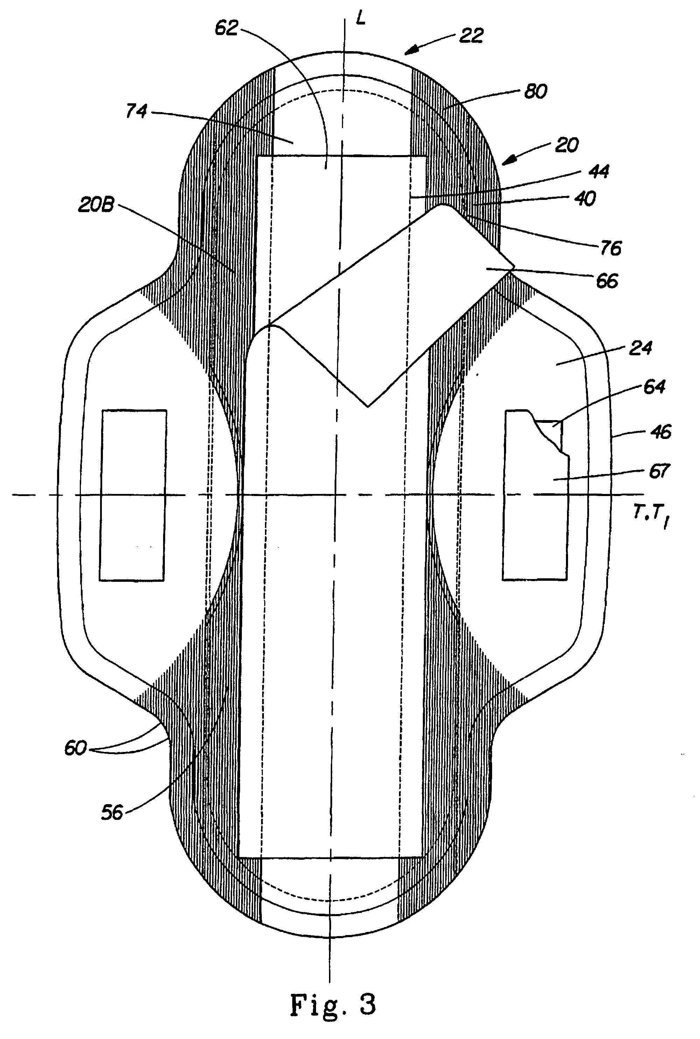

The garment surface 20B of the sanitary napkin 20 may include, and preferably does include, fasteners for attaching the sanitary napkin to the wearer's undergarment. Figure 3 shows the central pad fastener 62 which is adapted to secure the main body portion 22 of the sanitary napkin to the crotch region of an undergarment. Any types of fasteners known in the art can be used. Fasteners comprising adhesives have been found to work well for this purpose, with pressure-sensitive adhesives being preferred. Before the sanitary napkin 20 is placed in use, if an adhesive fastener is used, the adhesive is typically covered with a removable cover strip or release liner 66 in order to keep the adhesive from sticking to a surface other than the cortch portion of the panty prior to use. Suitable release liners are described in U.S. Patent 4,917,697.

Fig. 3 shows that the sanitary napkin 20 preferably also has a side flap fastener 64 on each side flap 24 which lies along the transverse centerline T of the sanitary napkin 20. The side flap adhesive fasteners 64 may be covered by separate removable release liners 67 to keep the flap adhesives 64 from sticking to extraneous surfaces prior to use. A suitable release liner that can be used for the side flap fasteners 64 is described in U.S. Patent Application Serial No, 08/247,912 filed May 23, 1994, entitled "Absorbent Article Having Flaps With Unitary Release Strip" in the name of Osborn, which was originally filed June 5, 1990 (PCT Publication No. WO 91/18574, published December 12, 1991). In other embodiments, the flaps 24 can have a unitary release member joined to a flap retaining member such as is described in U.S. Patent 5,520,676 issued to Lavash, et al. on May 28, 1996, for covering the flap adhesive. In other embodiments, the flaps 24 may comprise a unitary release material for the flap adhesive as described in PCT Publication No. WO 94/00093, published in the name of Lavash, et al. on January 6, 1994. In other embodiments, the central pad fastener 62 can be covered by an arrangement wherein the release liner 66 comprises a releasable wrapper that also serves as an individual package for the sanitary napkin. Suitable release liners that serve as an individual package for a sanitary napkin are described generally in U.S. Patent 4,556,146 issued to Swanson, et al. (which discloses a tri-folded sanitary napkin and wrapper).

Figure 4 shows an alternative embodiment of the sanitary napkin 20 of the present invention which has undergarment covering components (or "side wrapping elements") 50 that automatically wrap the sides of a wearer's undergarments when the undergarments are pulled up. The side wrapping elements 50 can be of any suitable size and shape. The side wrapping elements 50 each have a proximal edge 52 and a distal edge 54. The distal edges 54 of the side wrapping elements preferably extend outward beyond the longitudinal side edges 26 of the main body portion 22, a distance of less than or equal to about one-half the width of the main body portion 22.

The side wrapping elements 50 of the sanitary napkin 20 shown in FIG. 4 are preferably provided with the same type of pleated configuration, and many of the other features, including the zones of extensibility 56, that were described above in conjunction with the embodiment having side flaps. The side wrapping elements 50 preferably have a span from the distal edge of one side wrapping element 50 to the distal edge of the other side wrapping element of about 120 mm. The side wrapping elements 50 are preferably folded so that they are affixed about 40 mm inward from the distal edge 54 of the side wrapping element 50 and have a gap of between their proximal edges 52 of between about 30 - 60 mm, and more preferably, between about 40 - 50 mm.

The side wrapping elements 50 in this embodiment of the present invention may have any of the dimensions and characteristics set forth for the undergarment covering components in the aforementioned U.S. Patent Application Serial Nos. 08/096,121 and 08/124,180 filed in the names of Lavash, et al. and Mansfield, et al., and in allowed U.S. Patent Application Serial No. 08/277,733 filed in the name of Weinberger, et al. on July 20, 1994, all of which are incorporated by reference herein.

The zones of extensibility 56 in embodiments having side wrapping elements, are preferably capable of extending between about 20% or 30% and about 80%, more preferably between about 40% and about 70%, and most preferably between about 60% and about 70% under the forces associated with wearing the sanitary napkin in a pair of panties. Preferably, the zones of extensibility 56 are capable of such extension under forces of less than about 100 - 200 grams_{f} per inch (about 40-80 gf/cm), more preferably under forces of less than about 50 grams_{f} per inch (about 20gf/cm). Any inherent elasticity in the zones of extensibility 56 (that is, any tendency of the material comprising the zones of extensibility to return to its original dimension) is preferably relatively low to non-existent.

The zones of extensibility 56 should be located where the edges of the wearer's panties cross the distal edges 54 of the side wrapping elements 50 when the sanitary napkin 20 is placed in a pair of panties prior to the side wrapping elements 50 being folded around the edges of the panties. The distance between the two points where the panty edges cross the distal edges 54 of the side wrapping elements 50 varies depending on the size and style of panties. A representative distance between these two points is equal to about 85 mm. There are portions of the zones of extensibility 56 that are disposed longitudinally inboard of the points where the panty edges cross the distal edges 54 of the side wrapping elements 50 (that is, toward the transverse centerline T₁ of the side wrapping elements). In order to fit a wide variety of panty sizes and styles, it is preferred that each of the portions of the zones of extensibility between points where the panty edges cross the distal edges 54 of the side wrapping elements 50 is capable of extending greater than or equal to about 10-15 mm and that the combined extensibility in these portions for each side wrapping element is greater than or equal to about 20-30 mm. The longitudinal distance between the points within zones of extensibility 56 that are on opposite sides of the transverse centerline of a side wrapping element 50 is preferably between about 20 mm and about 150 mm, and more preferably is between about 30-130 mm, and most preferably between about 30-100 mm.

The side wrapping elements 50, as shown in FIG. 4, preferably also have a generally trapezoidally-shaped intermediate region or zone 58 located between the zones of extensibility 56. The intermediate region 58 preferably comprises a portion of the distal edges 54 of the side wrapping elements. This intermediate region 58 is stiffer (that is, more resistant to bending) than the zones of extensibility 56. The intermediate region 58 can also be less extensible than the portions of the side wrapping elements that comprise the zones of extensibility 56. The intermediate region 58 provides the side wrapping elements 50 with greater resistance to bending and crumpling so that the side wrapping elements will fold over the panty elastic, rather than crumple, when they are subject to compression by the wearer's thighs. The stiffer intermediate region 58 also helps to maintain panty elastic coverage when the wearer pulls her panties down to check the sanitary napkin for soiling, and then pulls her panties back up. The stiffer material helps to ensure that the side wrapping elements do not fold upward over top of the main body portion, and stay on the same plane as the main body portion of the sanitary napkin, where flimsier material would be pushed upward during the motion associated with pulling the panties downward and might tend to fold upward over the topsheet when the panties are pulled back up. Upon pulling the panty and sanitary napkin back up, flimsier material may also not be caught by the wearer's thighs and go back into place. The intermediate region 58 preferably has a distal edge portion that forms a portion of the distal edge 54 of the side wrapping elements. The length of the distal edge portion, is preferably at least about 20 mm, and more preferably about 30 mm.

The configuration and location of the zones of extensibility 56 in the embodiment shown in FIG. 4 is preferred for several reasons. The fact that the zones of extensibility 56 are spaced apart and separated by the stiffened intermediate region 58 provides improved resistance to bending and crumpling and more control over the manner of folding around the edges of the wearer's panties. The side wrapping elements 50 will typically fold in the zones of extensibility 56 and the intermediate region 58 between the points where the panty edges cross the distal edges 54 of the side wrapping elements 50. This makes the side wrapping elements sturdier and capable of more reliable folding than if the side wrapping elements were made entirely extensible and/or were made of materials having the same stiffness over their entire area.

In the alternative embodiment shown in FIG. 4, the portion of the topsheet forming the side wrapping elements 50 is formed into a Structural Elastic-Like Film (or "SELF") structure. Such a structure is described in greater detail in U.S. Patent 5,518,801 entitled "Web Materials Exhibiting Elastic-Like Behavior", issued to Chappell, et al. on May 21, 1996. The forming of a SELF structure into this portion of the topsheet provides the body-facing surface of the side wrapping elements 50 with improved softness so that it is comfortable for the wearer. In this embodiment, the SELFed portion of the topsheet is joined to an extension of a conventional inextensible backsheet by a layer of adhesive between these two components. The joinder to the relatively inextensible backsheet material effectively neutralizes the extensibility provided to the topsheet by the SELFing process. This is acceptable, however, since in this case the SELFing process is used only to increase the softness of the body-facing surface of the side wrapping elements 50.

The sanitary napkin 20 of the present invention is used by removing any release liner and thereafter placing the sanitary napkin 20 in a panty so that the central pad fastener 62 contacts the panty and maintains the sanitary napkin in position within the panty during use. In the case of embodiments having side flaps 24, the flap adhesive cover 67 is removed from the flap adhesive, and the flaps 24 are folded around the edges of the crotch region of the wearer's undergarments and attached either to the underside of the crotch region, or to the opposing flap. In the case of embodiments having side wrapping elements 50, the side wrapping elements 50 automatically wrap around the sides of the wearer's panties by the simple action of the wearer pulling up her panties.

The folded or pleated construction of the side flaps or side wrapping elements of the present invention provides several advantages. The securement of portions of the ends of the flaps or side wrapping elements in the manner described herein, and the folding and attachment of the same under the absorbent core, beneath the plane of the body-facing side of the main body portion, helps prevent the side wrapping elements, particularly the ends of the side wrapping elements, from flipping upward over the topsheet and causing discomfort for the wearer and/or loss of coverage of the wearer's undergarment. The pleated construction of these embodiments, particularly those having side flaps with zones of differential extensibility, provides an expedient means for creating a structure where at least portions of the zones of differential extensibility are located beneath the absorbent core, to provide the zones of extensibility that function in the best manner possible for a variety of undergarment sizes and styles. The pleated construction of the flaps or side wrapping elements also decouples the function of the flaps and side wrapping elements from the compression of the absorbent core to reduce the undesirable effects on the flaps or side wrapping elements when the absorbent core of the absorbent article is compressed by the wearer's legs.

Numerous alternative embodiments of the present invention are possible. For example, each of the side flaps or side wrapping elements are preferably mirror images of each other, and are symmetrical about the longitudinal centerline. However, it should be understood that the shape and location of the side flaps and side wrapping elements described herein are those of preferred embodiments, and other embodiments are also possible. For example, while the side flaps 24 and side wrapping elements 50 are shown as extending from each longitudinal edge of the main body portion, there may only be one side flap or side wrapping element extending from one of the edges of the main body portion. Further, the side flaps 24 and side wrapping elements 50 may be offset along the longitudinal centerline more towards one end edge of the main body portion than the other.

In other embodiments, only one of the components of the sanitary napkin 20 that forms the side flaps 24 or side wrapping elements 50 may be pleated. For example, the sanitary napkin 20 shown in FIG. 2 could be modified so that in cross-section, the backsheet 40 extends directly outward from the plane defined by the bottom of the absorbent core 42, and does not extend upward to meet the topsheet 38 at a location that is approximately even with the middle of the absorbent core 42 as in the case of the embodiment shown in FIG. 2. In such an embodiment, only the topsheet 38 will be folded under the garment-facing side 20B of the sanitary napkin 20. The backsheet 40 will already be extending to form the garment-facing side 20B of the sanitary napkin 20, and will be joined to the topsheet 38 beginning at the point shown in FIG. 2 where the side flaps 24 are at their closest position to the longitudinal centerline L.

In addition, the side flaps 24 or side wrapping elements 50 can be constructed of numerous other materials, versions of materials described herein, or combination of such materials. For instance, in other embodiments, the portion of the topsheet that extends outward to form the side wrapping elements 50 may be the apertured formed film described herein that is not formed into a "SELF" structure as the alternative embodiment shown in FIG. 4. In other embodiments, the portions of the topsheet and backsheet that form the side wrapping elements 50 can have one or more additional layers placed thereon, or therebetween, to provide the side wrapping elements 50 with greater resistance to crumpling by the wearer's thighs. In one version of such an embodiment, the side wrapping elements can comprise a laminate of the SELFed apertured formed film and polyethylene backsheet material described herein, which has an additional layer of apertured formed film disposed between the SELFed apertured formed film and polyethylene backsheet material.

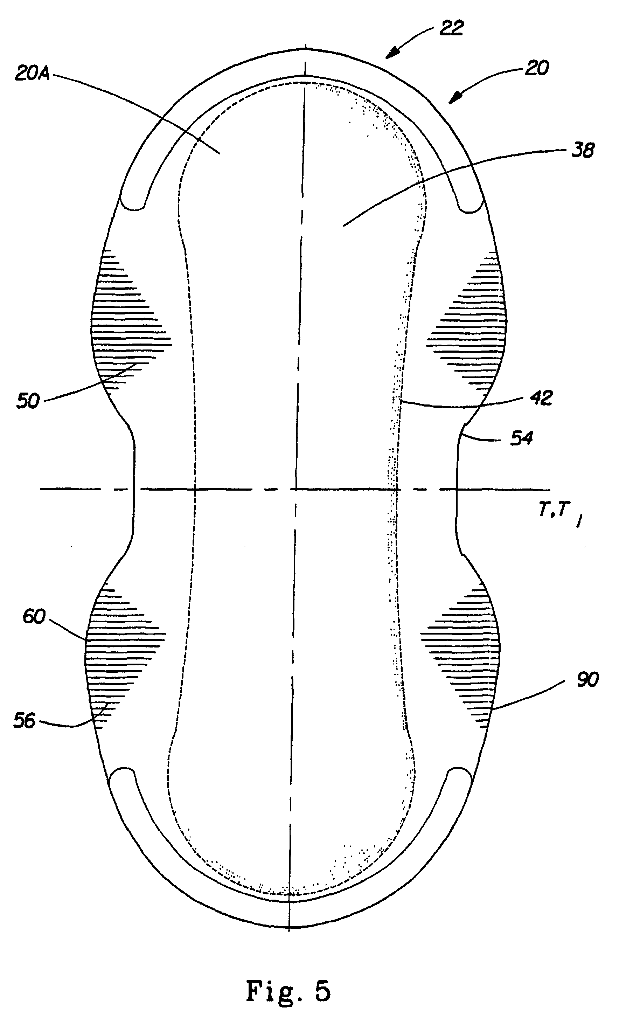

FIG. 5 shows another embodiment of the sanitary napkin 20 of the present invention having side wrapping elements 50. The sanitary napkin 20 is constructed similarly to the sanitary napkin shown in FIG. 4, in that the side wrapping elements 50 are folded under and attached to the garment-facing side of the main body portion 22. However, in the embodiment shown in FIG. 5, the side wrapping elements 50 are in an alternative configuration. In the embodiment shown in FIG. 5, the side wrapping elements 50 are each provided with two convex portions 90. The convex portions 90 are located on opposite sides of the side wrapping element transverse centerline T₁. The convex portions 90 are located in the same area as the zones of extensibility 56. This provides the sanitary napkin 20 with side wrapping elements 50 having additional material to cover the elasticized side edges of the wearer's panties at those places where the side wrapping elements 50 will intersect with the edges of the wearer's panties to provide improved protection from soiling.

The terms "panty liner" or "pantiliner" refer to absorbent articles that are less bulky than sanitary napkins which are generally worn by women between their menstrual periods. Suitable absorbent articles in the form of pantiliners that can be provided with the side flaps or side wrapping elements described herein are disclosed in U.S. Patent 4,738,676 entitled "Pantiliner" issued to Osborn on April 19, 1988.

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults or other incontinent persons. Suitable incontinent articles that can be provided with the side flaps or side wrapping elements described herein are disclosed in U.S. Patent 5,300,054 issued to Feist, et al. on April 5, 1994 and U.S. Patent 5,304,161 issued to Noel, et al. April 19, 1994.

The disclosures of all patents, patent applications (and any patents which issue thereon, as well as any corresponding published foreign patent applications), and publications mentioned throughout this patent application are hereby incorporated by reference herein. It is expressly not admitted, however, that any of the documents incorporated by reference herein teach or disclose the present invention. It is also expressly not admitted that any of the commercially available materials or products described herein teach or disclose the present invention.

## Claims

1. An absorbent article (20) for wearing in a wearer's undergarment that has a crotch region with a pair of side edges, said absorbent article (20) having a longitudinal centerline, a longitudinal dimension extending in a longitudinal direction, and a transverse dimension extending in a transverse direction, said absorbent article (20) comprising:
a main body portion (22) comprising an absorbent core (42), said main body portion (22) having a body-facing side, a garment-facing side (22B), and a pair of longitudinal side edges (26), said main body portion (22) comprising a liquid pervious topsheet (38), a liquid impervious backsheet (40) joined to said topsheet (38), and an absorbent core (42) positioned between said topsheet (38) and said backsheet (40); said absorbent article (20) further comprises
a pair of side flaps (24) for folding around the side edges of the wearer's undergarment or side wrapping elements (50) that automatically wrap the side edges of the wearer's undergarment, said side flaps (24) or side wrapping elements (50) having a transverse centerline T₁, said side flaps (24) or side wrapping elements (50) being comprised of an extension of at least one of said topsheet (38) and said backsheet (20) of said main body portion (22), said extension having two folds therein, said folds comprising a first fold (68) wherein said extension is folded inward toward said longitudinal centerline L under said garment-facing side (22B) of said main body portion (22) and is joined thereto at a first joined area, and a second fold (74) wherein said extension is folded outward away from the first joined area, said side flaps (24) or side wrapping elements (50) comprising longitudinal end regions (80) spaced longitudinally away from said transverse centerline T₁ and being positioned directly below the longitudinal side edges (26) of said main body portion (22), wherein said longitudinal end regions (80) of said side flaps (24) or side wrapping elements (50) are joined to the garment-facing side (22B) of said main body portion (22), and at least one of said side flaps (24) or side wrapping elements (50) comprises at least one zone of extensibility (56) and an adjacent region that is less extensible than said at least one zone of extensibility (56), **characterised in that** the portion of said extension of the second fold in a region of said transverse centerline T₁ extends freely laterally outward from said second fold (74) beyond the longitudinal side edges (26) of said main body portion (22) to distal edges (46).

2. The absorbent article (20) of Claim 1 wherein said at least one zone of extensibility (56) is primarily extensible in the longitudinal direction.

3. The absorbent article (20) of Claim 1 wherein said at least one zone of extensibility (56) is primarily extensible in the transverse direction.

4. The absorbent article (20) of Claim 1 wherein said at least one zone of extensibility (56) is extensible in a direction between the longitudinal direction and the transverse direction.

5. An absorbent article (20) according to any of the preceding claims wherein at least one zone of extensibility (56) comprises a region of said side flap (24) or side wrapping element (50) that is provided with corrugations having fold lines (60) therein.

6. An absorbent article (20) according to any of the preceding claims wherein when said absorbent article (20) comprises side flaps (24), said zones of extensibility (56) of said side flaps (24) are extensible in amounts greater than or equal to about 40% and less than or equal to about 150% under forces of less than about 200 grams force per inch, and when said absorbent article (20) comprises side wrapping elements (50), said zones of extensibility (56) of said side wrapping elements (50) are extensible in amounts greater than or equal to about 30% and less than or equal to about 80% under forces of less than about 200 grams force per inch.

7. The absorbent article (20) of Claim 6 wherein when said absorbent article (20) comprises side flaps (24), said zones of extensibility (56) of said side flaps (24) are extensible between about 70% and 120% under such forces, and when said absorbent article (20) comprises side wrapping elements (50), said zones of extensibility (56) of said side wrapping elements (50) are extensible between about 40% and 70% under such forces.

8. An absorbent article (20) according to any of the preceding claims wherein said side flaps (24) or side wrapping elements (50) comprise an extension of both said topsheet (38) and said backsheet (40).

9. An absorbent article (20) according to any of the preceding claims wherein only said extension of said topsheet (38) is folded inward toward the longitudinal centerline L under said garment-facing side (22B) of said main body portion (22), and a remaining portion of said topsheet (38) extends freely laterally outward from said first joined area, and said backsheet (40) is substantially free of folds and said backsheet (40) is joined to said remaining portion of said topsheet (38).

10. An absorbent article (20) according to any of the preceding claims wherein at least a portion of said at least one of zone of extensibility (56) is located below said absorbent core (42).

## Patentansprüche

1. Absorbierender Artikel (20) zum Tragen in der Unterwäsche eines Trägers, der eine Schrittregion mit einem Paar Seitenrändern hat, wobei der absorbierende Artikel (20) eine längs verlaufende Mittellinie L, eine sich in einer Längsrichtung erstreckende Abmessung und eine sich in einer Querrichtung erstreckende Querabmessung hat, wobei der absorbierende Artikel (20) aufweist:
einen Hauptkörperbereich (22) mit einem absorbierenden Kern (42), wobei der Hauptkörperbereich (22) aufweist eine körperseitige Seite, eine wäscheseitige Seite (22B) und ein Paar längs verlaufende Seitenränder (26), wobei der Hauptkörperbereich (22) aufweist eine flüssigkeitsdurchlässige Oberschicht (38), eine flüssigkeitsundurchlässige Unterschicht (40), die mit der Oberschicht (38) verbunden ist, und einen absorbierenden Kern (42), der zwischen der Oberschicht (38) und der Unterschicht (40) positioniert ist; wobei der absorbierende Artikel (20) ferner umfaßt
ein Paar Seitenklappen (24) zum Herumfalten um die Seitenränder der Unterwäsche des Trägers oder Seitenhüllelemente (50), die automatisch die Seitenränder der Unterwäsche des Trägers einhüllen, wobei die Seitenklappen (24) oder Seitenhüllelemente (50) eine quer verlaufende Mittellinie T1 haben, wobei die Seitenklappen (24) oder Seitenhüllelemente (50) gebildet werden aus einer Erstreckung wenigstens einer der Oberschicht (38) und der Unterschicht (40) des Hauptkörperbereichs (22), wobei die Erstreckung zwei darin ausgebildete Falten hat, wobei die Falten aufweisen eine erste Falte (68), in welcher die Erstreckung nach innen zu der längs verlaufenden Mittellinie L hin, unter die wäscheseitige Seite (22B) des Hauptkörperbereichs (22) gefaltet ist und mit diesem an einer ersten verbundenen Fläche verbunden ist, und eine zweite Falte (74), in welcher die Erstreckung von dem ersten verbundenen Bereich weg nach außen gefaltet ist, wobei die Seitenklappen (24) oder Seitenhüllelemente (50) längs verlaufende Endregionen (80) aufweisen, die in Längsrichtung weg von der quer verlaufenden Mittellinie T1 in Abstand liegen und direkt unterhalb der längs verlaufenden Seitenränder (26) des Hauptkörperbereichs (22) positioniert sind, wobei die längs verlaufenden Endregionen (80) der Seitenklappen (24) oder Seitenhüllelemente (50) mit der wäscheseitigen Seite (22B) des Hauptkörperbereichs (22) verbunden sind, und wenigstens eine der Seitenklappen (24) oder Seitenhüllelemente (50) wenigstens eine Dehnbarkeitszone (56) und eine angrenzende Region, die weniger dehnbar ist, als die wenigstens eine Dehnbarkeitszone (56) aufweist, **dadurch gekennzeichnet, daß** sich der Bereich der Dehnbarkeit der zweiten Falte in einer Region der quer verlaufenden Mittellinie T1 von der zweiten Falte (74) aus über die längs verlaufenden Seitenränder (26) des Hauptkörperbereichs (22) zu den distalen Rändern (26) seitlich frei nach außen erstreckt.

2. Absorbierender Artikel (20) nach Anspruch 1, in welchem wenigstens eine Dehnbarkeitszone (56) primär in der Längsrichtung dehnbar ist.

3. Absorbierender Artikel (20) nach Anspruch 1, in welchem wenigstens eine Dehnbarkeitszone (56) primär in der Querrichtung dehnbar ist.

4. Absorbierender Artikel (20) nach Anspruch 1, in welchem wenigstens eine Dehnbarkeitszone (56) in einer Richtung zwischen der Längsrichtung und der Querrichtung dehnbar ist.

5. Absorbierender Artikel (20) nach einem der vorstehenden Ansprüche, in welchem wenigstens eine Dehnbarkeitszone (56) eine Region der Seitenklappe (24) oder des Seitenhüllelements (50) aufweist, das mit Riffelungen versehen ist, die darin Faltlinien (60) haben.

6. Absorbierender Artikel (20) nach einem der vorstehenden Ansprüche, in welchem, wenn der absorbierende Artikel (20) Seitenklappen (24) aufweist, die Dehnbarkeitszonen (56) der Seitenklappen (24) unter Kräften von weniger als etwa 200 Gramm Kraft pro Inch in Beträgen dehnbar sind, die größer oder gleich etwa 40% und kleiner oder gleich etwa 150% betragen, und wenn der absorbierende Artikel (20) Seitenhüllelemente (50) umfaßt, die Dehnbarkeitszonen (56) der Seitenhüllelemente (50) unter Kräften von weniger als etwa 200 Gramm Kraft pro Inch in Beträgen dehnbar sind, die größer oder gleich etwa 30% und weniger oder gleich etwa 80% betragen.

7. Absorbierender Artikel (20) nach Anspruch 6, in welchem, wenn der absorbierende Artikel (20) Seitenklappen (24) aufweist, die Dehnbarkeitszonen (56) der Seitenklappen (24) unter solchen Kräften zwischen etwa 70% und 120% dehnbar sind, und wenn der absorbierende Artikel (20) Seitenhüllelemente (50) umfaßt, die Dehnbarkeitszonen (56) der Seitenhüllelemente (50) unter solchen Kräften zwischen etwa 40% und 70% dehnbar sind.

8. Absorbierender Artikel (20) nach einem der vorstehenden Ansprüche, in welchem die Klappen (24) oder Seitenhüllelemente (50) eine Erstreckung sowohl der Oberschicht (38) als auch der Unterschicht (40) umfassen.

9. Absorbierender Artikel (20) nach einem der vorstehenden Ansprüche, in welchem nur eine Erstreckung der Oberschicht (38) nach innen zu der längs verlaufenden Mittellinie L hin, unter die wäscheseitige Seite (22B) des Hauptkörperbereichs (22) gefaltet ist und sich ein verbleibender Bereich der Oberschicht (38) von dem ersten verbunden Bereich seitlich frei nach außen erstreckt und die Unterschicht (40) im wesentlichen faltenfrei ist und die Unterschicht (40) mit dem verbleibenden Bereich der Oberschicht (38) verbunden ist.

10. Absorbierender Artikel (20) nach einem der vorstehenden Ansprüche, in welchem wenigstens ein Bereich der wenigstens einen Dehnbarkeitszone (56) unter dem absorbierenden Kern (42) angeordnet ist.

## Revendications

1. Article absorbant (20) destiné à être porté dans un sous-vêtement d'utilisateur ayant une région d'entrejambe avec une paire de bords latéraux, ledit article absorbant (20) ayant une ligne médiane longitudinale (L), une dimension longitudinale s'étendant dans la direction longitudinale, et une dimension transversale s'étendant dans une direction transversale, ledit article absorbant (20) comprenant :
une partie de corps principale (22) comprenant une âme absorbante (42), ladite partie de corps principale (22) ayant une face côté corps, une face côté vêtement (22B), et une paire de bords latéraux longitudinaux (26), ladite partie de corps principale (22) comprenant une feuille de dessus (38) perméable aux liquides, une feuille de fond (40) imperméable aux liquides, réunie à ladite feuille de dessus (38), et une âme absorbante (42) placée entre ladite feuille de dessus (38) et ladite feuille de fond (40), ledit article absorbant (20) comprenant, en outre :
une paire de rabats latéraux (24) destinés à être repliés autour des bords latéraux du sous-vêtement de l'utilisateur ou une paire d'éléments d'enveloppement latéraux (50) qui enveloppent automatiquement les bords latéraux du sous-vêtement de l'utilisateur ; lesdits rabats latéraux (24) ou éléments d'enveloppement latéraux (50) ayant une ligne médiane transversale (T₁), et étant constitués d'une extension d'au moins l'une desdites feuille de dessus (38) et feuille de fond (40) de ladite partie de corps principale (22) ; ladite extension contenant deux plis ; lesdits plis comprenant un premier pli (68) ladite extension étant repliée vers l'intérieur, en direction de ladite ligne médiane longitudinale (L), sous ladite face côté vêtement (22B) de ladite partie de corps principale (22), et est réunie à celle-ci au niveau d'une première région réunie, et un deuxième pli (74) ladite extension étant repliée vers l'extérieur, en s'éloignant de la première région réunie ; lesdits rabats latéraux (24) ou éléments d'enveloppement latéraux (50) comprenant des régions d'extrémité longitudinales (80) espacées dans la direction longitudinale de ladite ligne médiane transversale (T₁) et positionnées directement en dessous des bords latéraux longitudinaux (26) de ladite partie de corps principale (22), lesdits régions d'extrémité longitudinales (80) desdits premiers rabats latéraux (24) ou éléments d'enveloppement latéraux (50) étant réunies à la face côté vêtement (22B) de ladite partie de corps principale (22) ; et au moins l'un desdits rabats latéraux (24) ou éléments d'enveloppement latéraux (50) comprenant au moins une zone d'extensibilité (56) et une région adjacente qui est moins extensible que ladite zone d'extensibilité (56) au moins, **caractérisé en ce que** la partie de ladite extension du deuxième pli, dans une région de ladite ligne médiane transversale (T₁), s'étend librement en s'éloignant l'extérieur dudit deuxième pli (74) dans la direction latérale, au-delà des bords latéraux longitudinaux (26) de ladite partie de corps principale (22), jusqu'à des bords distaux (46).

2. Article absorbant (20) selon la revendication 1, dans lequel ladite zone d'extensibilité (56) au moins est principalement extensible dans la direction longitudinale.

3. Article absorbant (20) selon la revendication 1, dans lequel ladite zone d'extensibilité (56) au moins est principalement extensible dans la direction transversale.

4. Article absorbant (20) selon la revendication 1, dans lequel ladite zone d'extensibilité (56) au moins est extensible dans une direction située entre la direction longitudinale et la direction transversale.

5. Article absorbant (20) selon l'une quelconque des revendications précédentes, dans lequel zone d'extensibilité (56) au moins comprend une région dudit rabat latéral (24) ou élément d'enveloppement latéral (50) qui est pourvue d'ondulations contenant des lignes de pliage (60).

6. Article absorbant (20) selon l'une quelconque des revendications précédentes, dans lequel, lorsque ledit article absorbant (20) comprend des rabats latéraux (24), lesdites zones d'extensibilité (56) desdits rabats latéraux (24) sont extensibles dans des proportions supérieures ou égales à environ 40 % et inférieures ou égales à environ 150 %, sous l'effet de forces inférieures à environ 80 g_{f}/cm (200 g_{f}/pouce), et, lorsque ledit article absorbant (20) comprend des éléments d'enveloppement latéraux (50), lesdites zones d'extensibilité (56) desdits éléments d'enveloppement latéraux (50) sont extensibles dans des proportions supérieures ou égales à environ 30 % et inférieures ou égales à environ 80 %, sous l'effet de forces inférieures à environ 80 g_{f}/cm (200 g_{f}/pouce).

7. Article absorbant (20) selon la revendication 6, dans lequel, lorsque ledit article absorbant (20) comprend des rabats latéraux (24), lesdites zones d'extensibilité (56) desdits rabats latéraux (24) sont extensibles dans des proportions comprises entre environ 70 % et 120 %, sous l'effet de ces forces, et, lorsque ledit article absorbant (20) comprend des éléments d'enveloppement latéraux (50), lesdites zones d'extensibilité (56) desdits éléments d'enveloppement latéraux (50) sont extensibles dans des proportions comprises entre environ 40 % et 70 %, sous l'effet de ces forces.

8. Article absorbant (20) selon l'une quelconque des revendications précédentes, dans lequel lesdits rabats latéraux (24) ou lesdits éléments d'enveloppement latéraux (50) comprennent une extension de ladite feuille de dessus (38) ainsi que de ladite feuille de fond (40).

9. Article absorbant (20) selon l'une quelconque des revendications précédentes, dans lequel seule ladite extension de ladite feuille de dessus (38) est repliée vers l'intérieur, en direction de la ligne médiane longitudinale (L), sous ladite face côté vêtement (22B) de ladite partie de corps principale (22), et la partie restante de ladite feuille de dessus (38) s'étend librement en s'éloignant de ladite première région de réunion, dans la direction latérale, et ladite feuille de fond (40) est essentiellement exempte de plis et est réunie à ladite partie restante de ladite feuille de dessus (38).

10. Article absorbant (20) selon l'une quelconque des revendications précédentes, dans lequel au moins une partie de ladite une zone d'extensibilité au moins (56) est située en dessous de ladite âme absorbante (42).
